# EUROPÄISCHE PATENTSCHRIFT

(11) **EP 3 790 443 B1**
(45) Veröffentlichungstag und Bekanntmachung des Hinweises auf die Patenterteilung: **28.06.2023**
(21) Anmeldenummer: 19722107.0
(22) Anmeldetag: 03.05.2019
(51) Int. Cl.: A61B 1/00, A61B 1/005, A61B 1/018

(54) **ENDOSKOP MIT AUSFAHRBAREM ARBEITSKANAL**
ENDOSCOPE WITH EXTENSIBLE WORK CHANNEL
ENDOSCOPE COMPRENANT UN CANAL DE TRAVAIL EXTENSIBLE

(30) Priorität: 03.05.2018 DE 102018110624
(43) Veröffentlichungstag der Anmeldung: 17.03.2021
(73) Patentinhaber: Bob, Konstantin, 69469 Weinheim (DE)
(72) Erfinder: Bob, Konstantin, 69469 Weinheim (DE)
(74) Vertreter: Winter, Brandl - Partnerschaft mbB
(86) Internationale Anmeldenummer: PCT/EP2019/061421
(87) Internationale Veröffentlichungsnummer: WO 2019/211457

(56) Entgegenhaltungen:
- DE-A1- 2 800 362
- DE-A1-102015 113 016
- DE-B2- 2 800 362
- US-A- 5 343 853
- US-A- 6 152 870
- US-A1- 2007 208 219
- US-A1- 2010 228 086

## Beschreibung

### Technisches Gebiet

Die vorliegende Offenbarung betrifft eine Arbeitskanaleinrichtung eines Endoskops sowie ein Endoskop und einen (Endoskop-separaten) Endoskopkopf-Aufsatz mit einer solchen Arbeitskanaleinrichtung.

### Hintergrund der Erfindung

Endoskope sind medizinische Arbeitsgeräte zur visuellen Exploration von Hohlräumen in einem Patientenkörper. Sie weisen grundsätzlich optische Einrichtungen am distalen, d.h. körperzugewandten Endoskopende (auch Endoskopkopf genannt) sowie optional einen Arbeitskanal auf, der sich ausgehend von einem proximalen (körperabgewandten) Endoskopabschnitt oder extrakorporalen Endoskopgriff durch einen (daran sich anschließenden) flexiblen/biegesteifen oder starren Endoskopschaft hindurch bis zum Endoskopkopf erstreckt und das extrakorporale Einführen und Verwenden eines medizinischen Instruments wie z.B. einer Zange, Schere, Nadel, Schlinge, Messer und dergleichen ermöglicht.

Derartige Endoskope können wahlweise mit zusätzlichen Fähigkeiten versehen werden, etwa indem am distalen Endoskopende/Endoskopkopf eine Kappe oder Hülse auf den Endoskopkopf radial außenseitig aufgesetzt wird, die mit bestimmten Funktionen/Funktionselementen versehen oder ausgerüstet ist, wodurch das Endoskop nicht nur zur Exploration und/oder als Zugang für therapeutische Anwendungen sondern auch selbst als ein minimalinvasives Instrument zur Ausführung eines chirurgischen Vorgangs genutzt werden kann. Alternativ hierzu ist es aber auch vorgesehen, spezielle Endoskope für ganz bestimmte medizinische Anwendungen mit derartigen Fähigkeiten integral auszustatten, wobei derartige Spezialanfertigungen jedoch nur für diese besondere Anwendung geeignet sind.

Verschiedene diagnostische und/oder therapeutische Verfahren erfordern beispielsweise eine Bildgebung und/oder bei Bedarf therapeutische Techniken am Gallen- und/oder Bauchspeicheldrüsengang sowie den Lebergängen des Patienten. Da die Vatersche Papille, welche den gemeinsamen Austritt von Gallen- und Bauchspeicheldrüsengang in das Duodenum bildet, seitlich in das Duodenum hineinragt, sind herkömmliche prograde (in Endoskoplängsrichtung blickende) Endoskope für derartige Eingriffe ungeeignet, da nicht genügend Schwenkraum im engen Duodenum (Durchmesser 3 bis 4 cm) vorhanden ist, um deren prograde Optik sowie den Arbeitskanal in eine seitwärts blickende Position auszurichten, da ein typischer Biegedurchmesser solcher Geräte bei etwa 12 cm liegt.

### Stand der Technik

Aus dem Stand der Technik (bspw. der US 2010/228086 A) sind zu diesem Zweck speziell angefertigte Duodenoskope bekannt, welche eine laterale (seitwärts blickende) oder retrograde (rückblickende) Optik (auch "Seitoptik" genannt) sowie einen seitwärts gerichteten Arbeitskanal aufweisen. Am Ausgang des Arbeitskanals solcher Duodenoskope ist zudem in der Regel ein sogenannter Albarranhebel vorgesehen, der durch Verschwenken ein gezieltes Führen/Umlenken eines im Arbeitskanal geführten Werkzeugs ermöglicht. Durch die seitwärts gerichtete Anordnung der Funktionseinheiten am Endoskopkopf wird eine Bildgebung und Behandlung im Bereich des Duodenums unter optimaler Nutzung des zur Verfügung stehenden Raums ermöglicht. Weitere Dokumente, die diesen Stand der Technik beschreiben, sind die Schriften US 6 152 870 A, US 2007/208219 A1, US 5 343 853 A, DE 28 00 362 A1 und DE 10 2015 113016 A1.

Derartige Endoskope mit Seitoptik sind jedoch sehr aufwendig und teuer in ihrer Fertigung und werden deshalb bislang als wiederverwendbare Geräte entwickelt und hergestellt. Der gekrümmte Arbeitskanal solcher Endoskope sowie die komplexe und hinterschnittreiche Konstruktion des Albarranhebels haben sich in der Praxis zudem als schwer sterilisierbar erwiesen bzw. der Sterilisationsprozess hat sich als zu materialermüdend für die empfindlichen Geräte herausgestellt, sodass nach einem Eingriff mit einem solchen Duodenoskop nur eine Desinfektion möglich ist. Dies hat zur Folge, dass nach einem Eingriff ein Bakterienrasen (Biofilm) im Arbeitskanal und/oder dem Hilfskanal des Endoskops bestehen bleiben kann. Löst sich dieser Biofilm dann bei einem darauf folgenden Eingriff, etwa weil ein Instrument durch den Arbeitskanal geschoben wird, so kann er bspw. in den Gallengang und/oder Pankreasgang gelangen und dort schwerwiegende Entzündungen bis hin zu einer Sepsis beim Patienten verursachen.

Weiter haben solche Geräte den Nachteil, dass sie nur für wenige, sehr spezifische Eingriffe im Bereich des Duodenums eingesetzt werden können, da weder die Optik noch der Arbeitskanal in prograde Richtung gerichtet werden können. Zudem ist die Navigation im Körper mit seitwärts blickenden Endoskopen im Allgemeinen eher schwierig, da ein Vorausblicken immer ein Abkrümmen des dem Endoskopkopf unmittelbar vorgeordneten "Deflectings" (aktiv abkrümmbarer Endoskopschaft-Abschnitt) um ca. 90° erfordert, wodurch wiederum mehr Platz in Endoskopquerrichtung benötigt wird, der nur im Magen vorhanden ist.

Das distale Deflecting des Schafts eines prograden (geradeausblickenden) flexiblen Endoskops mit abwinkelbarer Spitze besteht zumeist aus gelenkig verbundenen Ringelementen, welche die Stützstruktur des Schafts bilden und über Bowdenzüge, häufig Biegesteuerzüge genannt, bedient und gegeneinander gekippt werden. Um das Einführen in den Hohlraum zu erleichtern und das Eindringen von Substanzen zu verhindern, sind die Ringelemente von einer flexiblen Hülle aus einem Kunststoffmaterial umgeben. Im Inneren der Ringelemente verlaufen insbesondere Licht- und Bildleitkabel, Kanäle für Fluide oder endoskopische Arbeitsinstrumente. Die Biegesteuerzüge sind entlang der Außen- oder Innenseite der Ringelemente geführt. Derartige flexible Endoskope sind beispielsweise in US 6,270,453 B1, US 6,482,149 B1 oder DE 101 43 966 B4 offenbart.

Dabei ist der kleinste Radius, den der biegsame bzw. gelenkige Abschnitt des Schafts einnehmen kann, durch das jeweilige Konstruktionsprinzip vorgegeben. Beispielsweise erlaubt ein mit aufeinander folgenden, jeweils gelenkig miteinander verbundenen Ringelementen ausgebildeter Gelenkabschnitt nur einen relativ großen Biegeradius, da die einzelnen Ringelemente jeweils nur einen geringen Kippwinkel zum nächsten Ringelement aufweisen. Wenn die gelenkige Verbindung zweier Elemente nur eine Kippung um eine Achse ermöglicht, ist es für eine räumliche Biegemöglichkeit erforderlich, Elemente mit abwechselnd gegeneinander verdrehten Kippachsen anzuordnen, so dass nur jedes zweite Element in eine jeweils gewünschte Richtung auslenkbar ist; hierdurch wird der mögliche minimale Biegeradius nochmals vergrößert. Der Nahbereich neben dem Schaftende ist daher mit einer in der Endoskopspitze angeordneten Optik nicht einzusehen.

Aus dem Stand der Technik sind, wie bspw. in der DE 10 2013 222 279 A1 oder der DE 10 2012 220 578 A1 beschrieben, sind weiter Endoskope mit einer separat zum restlichen Endoskopkopf schwenkbaren Optik bekannt, welche sowohl in prograde als auch in seitliche Richtung blicken können. Solche Endoskope weisen aber keinen Arbeitskanal auf (werden also rein diagnostisch verwendet) oder weisen einen festen Arbeitskanal in prograder Richtung auf und sind somit nicht für die typischen Einsatzzwecke von Duodenoskopen geeignet, die einen seitwärts ausgerichteten Arbeitskanal voraussetzen.

Zusammengefasst kann man sagen, dass der Anwender mit den dem Stand der Technik bislang bekannten Endoskopen nicht in der Lage ist, sowohl in prograde Richtung als auch im seitlichen / rückblickenden Nahbereich des Endoskops minimalinvasive Eingriffe im Duodenum vorzunehmen.

Angesichts der vorstehend beschriebenen Nachteile des Stands der Technik, ist es die Aufgabe der vorliegenden Erfindung eine Arbeitskanaleinrichtung für ein Endoskop bereitzustellen, der eine integrierte minimalinvasive chirurgische Behandlung des die Endoskopspitze umgebenden Nahbereiches (sowohl lateral als auch prograd) ermöglicht.

### Kurzbeschreibung der Erfindung

Diese Aufgabe wird durch eine Arbeitskanaleinrichtung eines Endoskops gemäß Ansprüche 1 und 4 gelöst. Vorteilhafte Weiterbildungen sind Gegenstand der Unteransprüche.

Grundsätzlich betrifft die vorliegende Erfindung eine Arbeitskanaleinrichtung eines Endoskops zur Führung von medizinischen (minimalinvasiven) Werkzeugen und/oder zur Durchströmung von Medien. Die Arbeitskanaleinrichtung weist erfindungsgemäß einen Arbeitskanal auf, der zumindest abschnittsweise (mit seinem distalen Endabschnitt) biegeflexibel und relativ zum Endoskop ausfahrbar ausgebildet ist. Zumindest dieser distale Endabschnitt des Arbeitskanals (oder aber auch der gesamte Arbeitskanal) ist von einer ersten axial zurückgezogenen Stellung in eine zweite axial vorgeschobene Stellung überführbar bzw. verlagerbar/ausfahrbar. In der ersten Stellung ist der distale Arbeitskanalausgang in Axialrichtung des Endoskopkopfs (orthograd) ausgerichtet. In der zweiten, gegenüber der ersten Stellung in Richtung distal ausgefahrenen, Stellung kann der Arbeitskanalausgang unter Ausnutzung der Biegeflexibilität des distalen Endabschnitts mittels einer Führungseinrichtung in eine vorbestimmte laterale oder rückblickende Richtung umgelenkt werden oder wird bei der Ausfahrbewegung von der Führungseinrichtung zwangsweise/zwangsgeführt in die laterale Richtung umgelenkt. Die erfindungsgemäße Arbeitskanaleinrichtung erlaubt also ein Ausfahren und gleichzeitiges (oder verzögertes) Umlenken der distalen Arbeitskanalspitze und ermöglicht so die Behandlung sowohl in orthograder als auch in lateraler Richtung des Endoskopkopfes. Zusammengefasst betrifft die Erfindung eine Arbeitskanaleinrichtung mit einem Arbeitskanal dessen distale Spitze derart ausgebildet ist, dass sie ausfahrbar und (zum Suchen der Vaterschen Papille) zielgerichtet lenkbar ist.

Der Arbeitskanal ist mittels eines von der Handhabe her betätigbaren und im Bereich des Arbeitskanalausgangs am Arbeitskanal verankerten Schleppmittels, bspw. mittels eines Zugseils oder Bowdenzugs, von der ersten in die zweite Stellung schleppbar ausgebildet. Hierzu wird vorteilhafter Weise das Schleppmittel durch eine dem distalen Arbeitskanalende in der ersten Stellung distal vorgelagerte Umlenkeinrichtung (bspw. einer Umlenkkontur oder -rolle) umgelenkt, sodass der Arbeitskanal beim Aufbringen einer Zugkraft von der proximalen Richtung her zunächst gen distal geschleppt wird und anschließend der Umlenkung des Schleppmittels folgend in eine laterale Ausrichtung gegenüber der Endoskoplängsachse gezogen wird. Das Schleppmittel kann entweder manuell von der Bedienseite her oder kraftbetätigt mit einer Zugkraft beaufschlagt werden. Zur Rückführung kann eine Rückstellfeder oder dergleichen vorgesehen sein.

Gemäß einem bevorzugten, ggf. unabhängig zu beanspruchenden Ausführungsbeispiel kann die Führungseinrichtung eine konvexe Führungsfläche aufweisen, gegen welche der Arbeitskanal durch das Schleppmittel gezogen wird, wodurch die Führungsfläche den Arbeitskanal von radial innen abstützt und diesem eine definierte Krümmung verleiht. In anderen Worten kann sich der Arbeitskanal unter der in Proximalrichtung gerichteten Zugkraft des Schleppmittels gegen die Führungsfläche schmiegen und somit eine vordefiniert gekrümmte Form einnehmen. Die Führungseinrichtung kann hierbei bevorzugt auch das Schleppmittel umlenken. Besonders bevorzugt kann die Führungsfläche rinnenförmig oder als Nut ausgebildet sein, sodass der Arbeitskanal quer zu seiner Schlepprichtung abgestützt und in der Spur gehalten wird.

Gemäß einem bevorzugten, ggf. unabhängig zu beanspruchenden Ausführungsbeispiel kann der Arbeitskanal einen teleskopierbaren und / oder dehnbaren Verlängerungsabschnitt aufweisen, der sich bei einer Zugbelastung des Arbeitskanals in dessen Axialrichtung verlängert und dadurch die Ausfahrbewegung eines distalen Arbeitskanalabschnitts unterstützt. Auf diese Weise kann die Auflängung des Arbeitskanals auf einen definierten Bereich begrenzt werden, was die Reibung verringert. In alternativen Ausführungsformen kann der gesamte Arbeitskanal bei der Ausfahrbewegung sich relativ zum restlichen Endoskop verschieben oder die für die Ausfahrbewegung benötigte Länge wird aus der Elastizität/Dehnbarkeit der gesamten Länge des Arbeitskanals geholt.

Gemäß einer bevorzugten, ggf. unabhängig zu beanspruchenden Ausführungsform kann ein distaler Endoskopkopfabschnitt bzw. eine distale Endkappe des Endoskopkopfes in eine laterale Stellung schwenkbar sein. Der ausfahrbare bzw. ausziehbare Arbeitskanal kann in diesem Fall an dem besagten distalen Endoskopkopfabschnitt fixiert sein, sodass bei einer Schwenkbewegung des Endoskopkopfabschnitts dieser den Arbeitskanal mitschleppt, und dieser ausfährt. Da der Arbeitskanalausgang am schwenkbaren Endoskopkopfabschnitt winkelfest fixiert ist, wird dieser bei einem Verschwenken des Endoskopkopfabschnitts ebenfalls in eine laterale oder rückblickende Ausrichtung überführt.

Vorteilhafter Weise kann in einer solchen Ausführungsform bei einer Schwenkbewegung des distalen Endoskopkopfabschnitts eine Führungseinrichtung aufklappen, welche den ausgefahrenen Arbeitskanal von radial innen her mit einer definiert gekrümmten Kontur abstützt. Dies stellt sicher, dass der Arbeitskanal einen definierten Krümmungsverlauf einnimmt der die Durchführbarkeit von minimalinvasiven chirurgischen Instrumenten ermöglicht.

Gemäß einem bevorzugten, ggf. unabhängig zu beanspruchenden Aspekt der Erfindung kann der Arbeitskanal in einem Randbereich des Querschnitts des Endoskops angeordnet sein. D.h. der Arbeitskanal kann entweder innerhalb oder außerhalb (entlang) des Schafts und im Bereich des Außenumfangs geführt werden. Hierbei sollte die Arbeitskanaleinrichtung derart angepasst und ausgebildet sein, dass der distale Endabschnitt des Arbeitskanals derart umgelenkt wird, dass der Arbeitskanalausgang zu dem dem Arbeitskanal gegenüberliegenden Randbereich des Querschnitts des Endoskops hin ausgerichtet ist. In anderen Worten kann der Arbeitskanal bevorzugt im Randbereich des Endoskopquerschnitts angeordnet sein und beim Ausfahren derart umgelenkt werden, dass er sich über den Endoskopquerschnitt hinweg erstreckt. Auf diese Weise kann der Krümmungsdurchmesser beim Ausrichten in die laterale Richtung maximiert werden, was das Durchführen minimalinvasiver chirurgischer Instrumente erleichtert.

Besonders bevorzugt kann der Arbeitskanal am von der der Optik eines Endoskops abgewandten Randbereich angeordnet sein und im ausgefahrenen, durch die Führungseinrichtung umgelenkten Zustand derart umgelenkt werden, dass der Arbeitskanal in lateral und in Richtung hin zur Blickrichtung der Optik ausgerichtet ist.

Gemäß einer weiteren bevorzugten und ggf. unabhängig zu beanspruchenden Ausführungsform kann der Arbeitskanal von proximaler Richtung her schiebend antreibbar sein, um die Ausfahrbewegung zu bewirken. Bei einer solchen Ausführungsform muss der Arbeitskanal eine ausreichende Schubsteifigkeit aufweisen und über seine gesamte Länge relativverschiebbar an oder in dem Endoskopschaft angeordnet sein. Der Vorschub kann manuell oder kraftbetätigt erfolgen, bspw. mittels einer Hydraulik eines Elektromotors oder dergleichen.

Gemäß einer weiteren bevorzugten, ggf. unabhängig zu beanspruchenden Ausführungsform kann der Arbeitskanal außerhalb des Endoskopschafts angeordnet sein. Hierzu kann beispielsweise eine Lasche in einer Umhüllung des Endoskops ausgebildet sein, ähnlich wie bei einem Vorhang, oder es kann eine (elastisch) dehnbare Doppelung in der Endoskopumhüllung ausgebildet sein durch welche der Arbeitskanal entlang des Endoskopschafts bis zur Endoskopspitze geführt wird. Es können auch andere, separate Koppelelemente vorgesehen sein, bspw. kann ein Führungskanal für den ausfahrbaren Arbeitskanal in regelmäßigen Abständen entlang des Schafts mit Tape befestigt werden.

Gemäß einer weiteren bevorzugten, ggf. unabhängig zu beanspruchenden Ausführungsform kann der Arbeitskanal über einen eigenen (internen) aktiv abkrümmbaren Abschnitt verfügen. Bspw. kann ein Bowdenzug in der Wandung des ausfahrbaren Arbeitskanals verlaufen, sodass dieser im ausgefahrenen Zustand vom Griffteil her definiert abwinkelbar ist, um z.B. die Vatersche Papille zielgerichtet zu intubieren. In einer solchen Ausführungsform könnte also der Arbeitskanal autonom vom Endoskop vor- und zurückfahrbar sowie abwinkelbar ausgebildet sein.

Gemäß einer weiteren bevorzugten, ggf. unabhängig zu beanspruchenden Ausführungsform kann die Führungseinrichtung einen am distalen Endabschnitt des Arbeitskanals angeordneten Abschnitt aus einem Formgedächtnismaterial (-draht) aufweisen, mittels dessen in der zweiten, ausgefahrenen Stellung des Arbeitskanals eine definierte Krümmung bewirkt werden kann.

Gemäß einer weiteren bevorzugten, ggf. unabhängig zu beanspruchenden Ausführungsform kann die Wandung des distalen Arbeitskanalabschnitts verstärkt ausgeführt sein, um den beim Durchschieben minimalinvasiver chirurgischer Instrumente auftretenden Kräften besser standhalten zu können. Hierzu können bspw. Hülsen aus einem steifen Material oder ein Drahtgeflecht in den distalen Abschnitt des Arbeitskanals eingearbeitet werden.

Ein weiterer, ggf. unabhängig zu beanspruchender Aspekt betrifft ein Endoskop oder einen adaptiven Endoskopkopf mit einer Arbeitskanaleinrichtung, wie vorstehend beschrieben. Um die Umlenkbarkeit des Arbeitskanals optimal nutzen zu können und sowohl in orthograde als auch in laterale Richtung minimalinvasive chirurgische Behandlungen ausführen zu können, ist es zweckmäßig auch in beide dieser Richtungen eine Bildgebungsmöglichkeit bereitzustellen. Gemäß einer Ausführungsform kann das Endoskop bzw. der Endoskopkopf hierzu eine in Axialrichtung blickende Optik und eine zusätzliche lateral blickende Optik aufweist, oder dass die Optik von einer in Axialrichtung blickenden Ausrichtung in eine lateral blickende Ausrichtung überführbar ausgebildet ist.

Ein weiterer, unabhängiger Aspekt der Erfindung betrifft ein Endoskop, das zumindest eine Optik zur Bildübertragung; ein Leuchtmittel oder Lichtleiter und einen eigenen Arbeitskanal zur Führung von medizinischen Werkzeugen und/oder zur Durchströmung von Medien aufweist. Zumindest ein (distaler) Abschnitt des Arbeitskanals ist biegeflexibel sowie relativ zum Endoskop (in Axialrichtung) ausfahrbar ausgebildet. Durch die Ausfahrbarkeit in Kombination mit der Biegeflexibilität kann der Arbeitskanal(-abschnitt) von einer ersten Stellung, in der der Arbeitskanalausgang in Axialrichtung des Endoskopkopfs ausgerichtet ist, in eine zweite, gegenüber der ersten Stellung in Richtung distal ausgefahrene Stellung verlagert werden, in welcher der Arbeitskanalausgang mittels einer Führungseinrichtung in eine vorbestimmte laterale und/oder rückblickende Richtung umgelenkt/ausgerichtet wird.

Ein weiterer, unabhängiger Aspekt der Erfindung betrifft einen Endoskopkopf der Endoskop-adaptiven Bauart nach Art eines separaten, zusätzlichen Endoskopkopf-Aufsatzes, der ein Befestigungsmittel zur lösbaren Befestigung an dem Endoskopkopf eines Endoskops hat. Der erfindungsgemäße adaptive Endoskopkopf hat zumindest einer eigenen (separate) Optik zur Bildübertragung; ein eigenes (separates) Leuchtmittel oder einen eigenen (separaten) Lichtleiter; und einen eigenen Arbeitskanal zur Führung von medizinischen Werkzeugen und/oder zur Durchströmung von Medien. Der Arbeitskanal ist zumindest abschnittsweise biegeflexibel und relativ zum Endoskopkopf ausfahrbar ausgebildet und kann von einer ersten Stellung, in der der Arbeitskanalausgang in Axialrichtung des Endoskopkopfs ausgerichtet ist, in eine zweite, gegenüber der ersten Stellung in Richtung distal ausgefahrene Stellung verlagert/überführt werden. In der Ausgefahrenen Stellung kann der Arbeitskanalausgang erfindungsgemäß mittels einer Führungseinrichtung in eine laterale oder rückblickende Richtung umgelenkt/ausgerichtet werden.

### Kurzbeschreibung der Figuren

Fig. 1 ist eine Darstellung zur Veranschaulichung eines Anwendungsgebietes eines Endoskops mit einer erfindungsgemäßen Arbeitskanaleinrichtung;
Fig. 2 ist eine perspektivische Darstellung eines Endoskops gemäß einer ersten Ausführungsform der Erfindung mit orthograd ausgerichtetem Arbeitskanalausgang;
Fig. 3 ist eine perspektivische Darstellung eines Endoskops gemäß der ersten Ausführungsform der Erfindung mit ausgefahrenem, lateral ausgerichtetem Arbeitskanalausgang;
Fig. 4 ist eine perspektivische Darstellung eines Endoskops gemäß einer zweiten Ausführungsform der Erfindung mit orthograd ausgerichtetem Arbeitskanalausgang;
Fig. 5 ist eine perspektivische Darstellung eines Endoskops gemäß der zweiten Ausführungsform der Erfindung mit ausgefahrenem, lateral ausgerichtetem Arbeitskanalausgang;
Fig. 5A ist eine alternative Führungseinrichtung für einen ausfahrbaren Arbeitskanal;
Fig. 6 ist eine perspektivische Darstellung eines Endoskops gemäß einer dritten Ausführungsform der Erfindung mit orthograd ausgerichtetem Arbeitskanalausgang;
Fig. 7 ist eine perspektivische Darstellung eines Endoskops gemäß der dritten Ausführungsform der Erfindung mit ausgefahrenem, lateral ausgerichtetem Arbeitskanalausgang;
Fig. 8 zeigt eine weitere Ausführungsform mit schräger Optik;
Fig. 9 zeigt eine weitere Ausführungsform mit schwenkbarer Optik; und
Fig. 10 zeigt einen alternativen Verlängerungsabschnitt für einen ausfahrbaren Arbeitskanal.

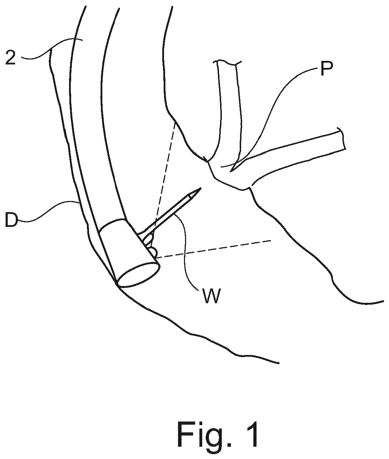

Fig. 1 Zeigt die Papilla Vateri (P), die im rückwärtigen (dorsalen) absteigenden Teil (Pars descendens) des Zwölffingerdarms (Duodenum) (D) lokalisiert und durch die gewundene Geometrie dieses Systems relativ schwer zugänglich ist. Der zur Verfügung stehende Raum im Bereich des Duodenums (D) ist sehr begrenzt, wodurch Eingriffe an der Papilla Vateri (P) mit herkömmlichen, prograden Endoskopen nicht möglich sind, da bei einem entsprechenden Abwinkeln der Endoskopspitze in Richtung Eingriffsabschnitt nicht mehr genügend Distanz zum Lumen des Duodenums (D) für eine ordentliche Bildgebung bliebe.

Aus diesem Grund sind dem Stand der Technik die eingangs genannten Duodenoskope bekannt, die eine fest seitwärts bzw. rückblickende Optik sowie einen entsprechend ausgerichteten Arbeitskanal aufweisen, um den zur Verfügung stehenden Raum optimal zu nutzen. Solche Duodenoskope haben jedoch den Nachteil, dass sie in ihrer lateralen/retrograden Ausrichtung der Optik und des Arbeitskanals festgelegt sind. Dies erschwert zum einen die allgemeine Navigation innerhalb des Patienten und macht solche Endoskope zum anderen unflexibel in ihren Einsatzmöglichkeiten. Es handelt sich in anderen Worten um teure Spezialgeräte für ein eng eingeschränktes Anwendungsgebiet.

Ein Grundgedanke der vorliegenden Erfindung ist es deshalb, einen Arbeitskanalmechanismus für ein Endoskop bereitzustellen, der von einer geradeaus blickenden Ausrichtung in eine laterale oder rückblickende Ausrichtung verlagerbar ist.

### Erstes Ausführungsbeispiel

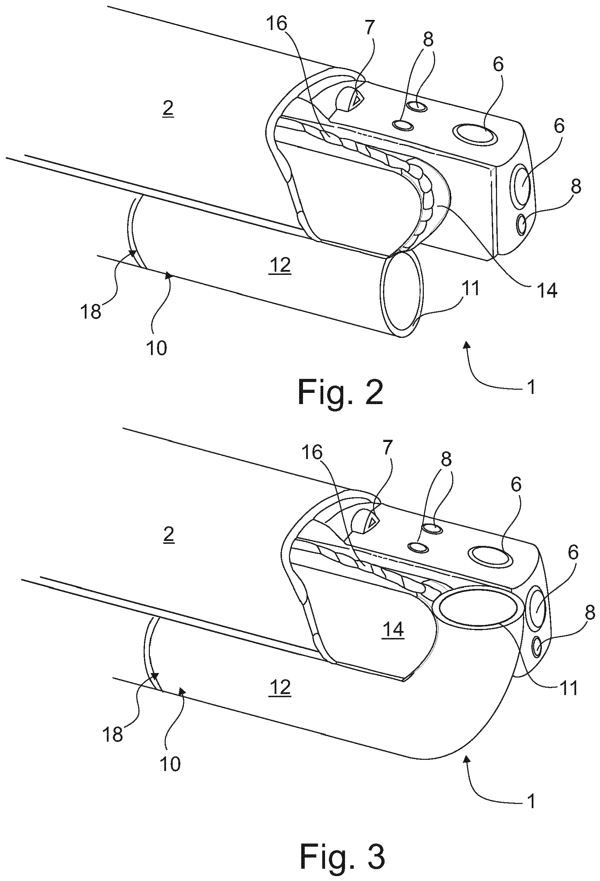

In Fig. 2 ist eine erste beispielhafte Ausführungsform eines erfindungsgemäßen Endoskops 2 dargestellt. Am distalen Ende des Endoskops 2 ist ein Endoskopkopf 4 angeordnet, der verschiedene Funktionseinheiten wie eine Optik 6; eine Reinigungsdüse 7 für das Objektiv der Optik 6 und Leuchtmittel 8 aufweist. Der Übersichtlichkeit halber sind in den Figuren nur die oben genannten nötigsten Funktionseinheiten dargestellt, selbstverständlich kann ein erfindungsgemäßer Endoskopkopf 4 zusätzlich diverse andere dem Stand der Technik bekannte Funktionseinheiten aufweisen, wie z.B. Absaugkanäle und dergleichen.

Ein Arbeitskanal 10 des Endoskops 2 erstreckt sich von seiner distalen Öffnung bzw. seinem Arbeitskanalausgang 11 im Bereich des distalen Endes des Endoskops 2, durch einen flexiblen Schaft bzw. entlang eines flexiblen Schaftes eines mit dem Endoskopkopf 4 bestückten Endoskops 2 hindurch, bis hin zu einer proximalen Öffnung im Bereich des Endoskopgriffs (nicht dargestellt) und kann so bspw. zum Einführen von chirurgischen Instrumenten oder Werkzeugen (W), wie einem Papillotom, oder zum Applizieren von Medien im Patienten verwendet werden. Das dargestellte Endoskop 2 weist weiter in seinem Innern verlaufende (nicht dargestellte) Funktions- und Versorgungskanäle, wie bspw. elektrische Leitungen, zum Versorgen der Funktionseinheiten, zur Übermittlung von Daten und zum Steuern der Bewegungen des Endoskops auf, die in proximaler Richtung an eine Betriebsstation (nicht dargestellt) bzw. einen Controller/ ein Steuergerät (ebenfalls nicht dargestellt) anschließbar sind. Darüber hinaus kann ein solches Endoskop der Schaftbauart zwischen dem Endoskopkopf 4 und dem vorzugsweise flexiblen Endoskopschaft ein sogenanntes Deflecting aufweisen, das in Verlängerung des passiv biegsamen Endoskopschafts einen aktiv abkrümmbaren Schaftabschnitt darstellt. Dieser aktiv abkrümmbare Schaftabschnitt kann entweder in alle Richtungen abkrümmbar sein oder ggf. nur in eine Richtung, wobei im letzteren Fall zwischen Deflecting und Endoskopschaft ein Rotationskranz angeordnet sein kann, der das Deflecting bezüglich des Endoskopschafts um die Endoskoplängsachse drehbar hält.

In der in den Figuren 2 und 3 dargestellten ersten Ausführungsform eines erfindungsgemäßen Endoskops ist der Arbeitskanal 10 ausfahrbar gehalten. Genauer gesagt ist ein distaler Endabschnitt 12 des Arbeitskanals 10 teleskopierbar gegenüber dem Arbeitskanalgrundkörper. Die Ausfahrbarkeit kann aber genauso gut auf eine andere Weise erzeugt werden, wie an anderer Stelle genauer erläutert wird. Der distale Endabschnitt 12 des Arbeitskanals 10 ist zudem biegeflexibel gehalten. Durch die Ausfahrbarkeit des Arbeitskanals 10 in Kombination mit der Biegeflexibilität wird erreicht, dass der Arbeitskanal 10 von der in Figur 2 dargestellten proximal zurückgezogenen und geradeausblickenden Stellung in die in Figur 3 dargestellte, gen distal vorgeschobene und umgelenkte Stellung überführt werden kann.

Das Ausfahren des Arbeitskanals wird in der ersten Ausführungsform durch ein Schleppmittel 16 (hier ein Seilzug) bewerkstelligt. Das Schleppmittel kann vom proximalen Griffteil her betätigt werden und schleppt den distalen Endabschnitt 12 des Arbeitskanals 10 in die lateral ausgerichtete Stellung. Hierbei fährt der teleskopierbare distale Endabschnitt 12 des Arbeitskanals 10 aus dem diesem proximal vorgelagerten Arbeitskanalabschnitt aus. Eine Führungseinrichtung 14 sorgt bei der Ausfahrbewegung dafür, dass die Umlenkung des Arbeitskanals auf definierte Weise erfolgt. Die Führungseinrichtung 14 ist in der ersten Ausführungsform der Erfindung als eine Art Rampe mit einer Führungsnut oder -rinne ausgebildet, die einen definierten Krümmungsverlauf für den distalen Endabschnitt 12 des Arbeitskanals 10 vorgibt. Die Führungseinrichtung 14 wölbt sich kuppelartig zum distalen Endoskopende hin. Die vom Schleppmittel 16 ausgeübte Zugkraft wird auf diese Weise am Anfang des Schleppvorgangs durch die Führungseinrichtung 14 um näherungsweise 180° umgelenkt, sodass der Arbeitskanal 10 gen distal nach vorne gezogen wird, und wirkt zum Ende der Schleppbewegung gen proximal, so dass der Arbeitskanal 10 gegen die Führungseinrichtung 14 gehalten wird und sich an diese anschmiegt. Dies gewährleistet einen definierten Krümmungsradius, durch welchen bspw. ein chirurgisches Instrument geschoben werden kann und verhindert zudem ein Abknicken beim Überführen des Arbeitskanals 10 von der orthograden in die laterale Ausrichtung.

Wie in den Figuren 2 und 3 gut zu erkennen ist, verläuft der Arbeitskanal 10 grundsätzlich auf der von der Optik 6 abgewandten Seite des Endoskops. Beim Ausfahren und Umlenken des distalen Endabschnitts 12 des Arbeitskanals 10, wird dieser derart von der Führungseinrichtung 14 umgelenkt, dass er in dieselbe Richtung wie die Optik 6 blickt. Auf diese Weise wird der Krümmungsradius des Arbeitskanals 10 im umgelenkten Zustand maximiert, was das Durchführen minimalinvasiver chirurgischer Werkzeuge erleichtert. Der Arbeitskanal 10 verläuft in dieser Ausführungsvariante außerhalb des Endoskopschafts, kann aber auch innerhalb desselben (im Randbereich) angeordnet sein, wie in einigen der folgenden Ausführungsbeispielen zu sehen ist.

Das Schleppmittel 16 bzw. der Seilzug der ersten Ausführungsform kann manuell bspw. über Dreh- oder Kniehebel betätigt werden oder auch mittels einem Elektromotor oder einer Hydraulik kraftbetätigt aktuiert werden.

Über den in den Figuren 2 und 3 dargestellten Endoskopkopf 4 wird im fertig montierten Zustand eine hier ausgeblendete Kappe angeordnet, die die Komponenten abschirmt und den Endoskopkopf 4 an seinem distalen Ende eine kontinuierliche glatte Kontur gibt, die das Einführen des Endoskops 2 erleichtert. Außerdem kann das Endoskop 2 der ersten und aller weiteren Ausführungsformen mit einer Schutzhülle überzogen werden, um das Endoskop 2 abzudichten und dessen Gleiteigenschaften zu verbessern.

### Zweites Ausführungsbeispiel

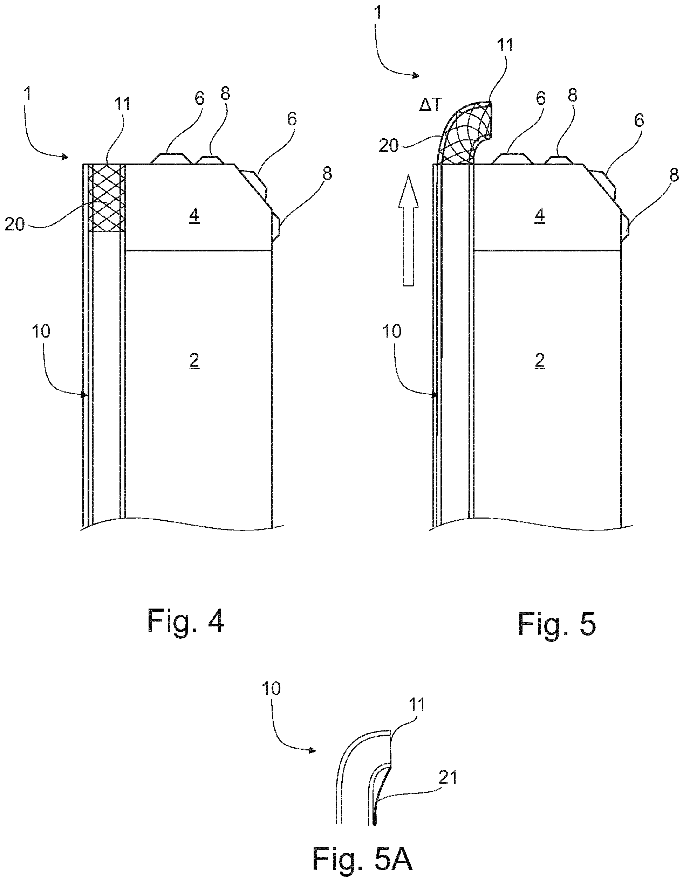

In der in den Figuren 4 und 5 dargestellten zweiten Ausführungsform ist der gesamte Arbeitskanal 10 relativ zum Endoskop in dessen Axialrichtung verschiebbar ausgeführt. Auf diese Weise kann proximal vom Griffteil her eine Vorschubbewegung erzeugt werden, die das Ausfahren des distalen Arbeitskanalendes bewirkt. Die Vorschubbewegung kann beispielsweise manuell über einen Dreh- oder Hebelmechanismus oder mittels einer Antriebseinheit (bspw. Linearmotor) aktuiert werden. Der Arbeitskanal 10 muss bei einer solchen Ausführungsform mit einer entsprechend hohen Schubsteifigkeit ausgelegt sein, dass die Vorschubbewegung bis zur distalen Endoskopspitze übertragen werden kann. In der zweiten Ausführungsform der Figuren 4 und 5 ist die Führungseinrichtung 20 als eine Art Korsett aus einem Formgedächtnismaterial ausgeführt. Im Vorgeschobenen Zustand kann durch eine Temperaturbeeinflussung vom Griffteil her ein vorbestimmter, abgekrümmter Zustand der Führungseinrichtung 20 bewirkt werden. Hierzu ist die Führungseinrichtung 20 wärmeleitend mit einer entsprechenden Temperaturbeeinflussungseinrichtung (nicht dargestellt) verbunden. Durch die korsettartige Führungseinrichtung wird der distale Endabschnitt 12 des Arbeitskanals 10 zudem versteift, sodass er ein Umlenken von durch diesen hindurchgeschobenen minimalinvasiven chirurgischen Instrumenten und Werkzeugen erzwingen kann.

Im zweiten Ausführungsbeispiel verläuft der Arbeitskanal 10 auf der von der Optik 6 abgewandten Seite des Endoskops 2 innerhalb des Endoskopschafts und wird von der Führungseinrichtung 20 in Richtung hin zur Blickrichtung der Optik 6 ausgelenkt, um eine Behandlung in lateraler Richtung zu ermöglichen.

Alternativ oder zusätzlich zu dem Formgedächtnismaterial kann, wie in Fig. 5A dargestellt, zumindest ein separater Bowdenzug 21 als Führungseinrichtung zur aktiven Abwinkelung des Arbeitskanals 10 verwendet werden. Hier verläuft der Bowdenzug 21 weitestgehend in der Wandung des Arbeitskanals 10, tritt in einem Bereich vor dem Arbeitskanalausgang 11 aus der Wandung aus und ist im Bereich des Arbeitskanalausgangs 11 an dem Arbeitskanal 10 verankert. Auf diese Weise lässt sich der distale Endabschnitt 12 des Arbeitskanals 10 autonom vom Griffteil her steuern (abwinkeln), bspw. mittels eines Hebels oder eines Elektroantriebs, um die Vatersche Papille zielgerichtet zu intubieren.

### Drittes Ausführungsbeispiel

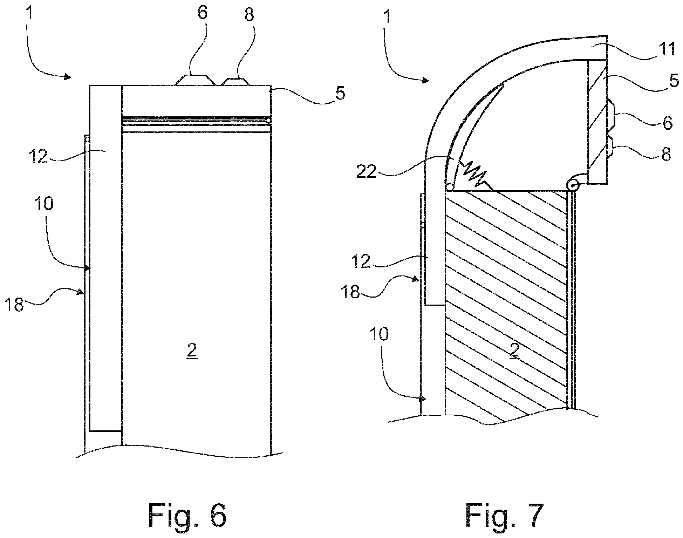

In einem dritten Ausführungsbeispiel, das in den Figuren 6 und 7 dargestellt ist, ist ein distaler Abschnitt des Endoskopkopfes 4 als eine schwenkbare Endkappe ausgeführt. Hierzu ist die schwenkbare Endkappe im gezeigten Beispiel an einem Scharnier im Randbereich des Endoskopkopfes 4 gelenkig gelagert und kann, bspw. über einen Bowdenzug, zum Verschwenken betätigt werden. Der distale Endabschnitt 12 des Arbeitskanals 10 ist hierbei an dem verschwenkbaren Endabschnitt des Endoskopkopfes verankert und wird beim Verschwenken desselben mitgeschleppt.

Um mit einem erfindungsgemäßen Endoskop sowohl in orthograder als auch in lateraler Richtung operieren zu können, ist es wichtig, in beide dieser Richtungen auch eine Möglichkeit zur Bildgebung zu haben. In der in den Figuren 2 und 3 dargestellten ersten Ausführungsform sind Optik 6 und Leuchtmittel 7 hierzu jeweils doppelt ausgeführt, einmal in orthograde Richtung ausgerichtet und einmal in laterale Richtung ausgerichtet. Der Anwender kann so zwischen diesen beiden Bildgebungen hin und her schalten oder beide gleichzeitig (z.B. auf verschiedenen Bildschirmen) ausgeben lassen, je nach Bedarf.

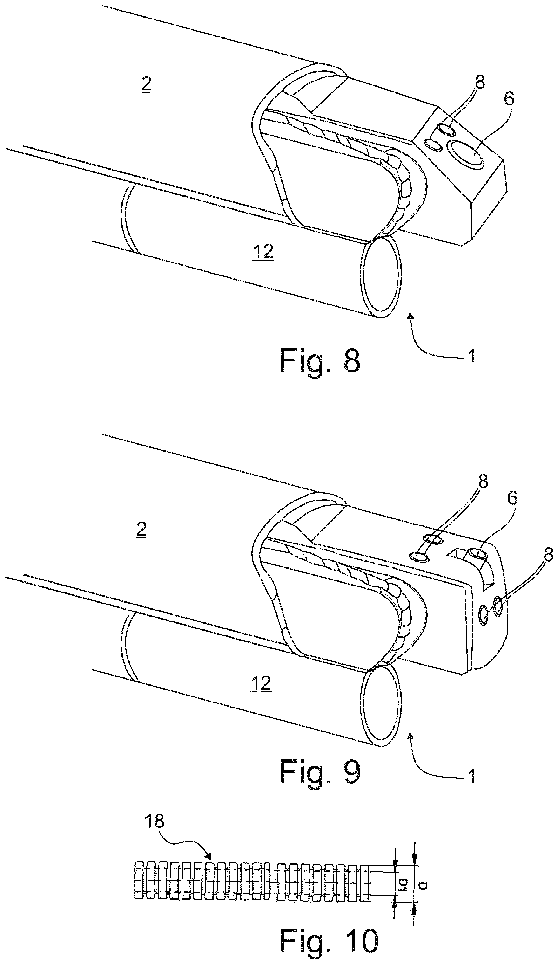

Eine in Figur 8 dargestellte Variante einer vorteilhaften Bildgebungseinrichtung verwendet eine einzige, schräg (zwischen orthograd und lateral) ausgerichtete Optik 6. Durch die Verwendung einer Optik 6 mit ausreichend großem Blickwinkel können auf diese Weise (chirurgische) Arbeiten sowohl in orthograder als auch in lateraler Stellung des Arbeitskanals 10 ausgeführt werden. Bevorzugt kann die Optik eines erfindungsgemäßen Endoskops einen Blickwinkel zwischen 115° und 140° ermöglichen.

In einer weiteren, in der Figur 9 dargestellten, Ausführungsvariante ist die Optik 6 schwenkbar ausgeführt. Vergleichbar mit einem Teleskop in einer Sternwarte kann das Objektiv leicht aus der Oberfläche des Endoskopkopfes 4 hervorstehen und zwischen einer orthograden und einer lateralen Ausrichtung schwenkbar ausgebildet sein. Zum Verschwenken der Optik kann bspw. ein Bowdenzug, ein Kettentrieb oder dergleichen eingesetzt werden. Bei entsprechend großem Blickwinkel ist es auch ausreichend, wenn die Optik 6 in einem Bereich zwischen diesen beiden Endpositionen verschwenken kann.

Wie bereits angedeutet, gibt es mehrere Möglichkeiten, die Ausfahrbarkeit des Arbeitskanals zu bewerkstelligen. Bereits beschrieben wurde ein teleskopartiger Abschnitt, der bspw. in der Fig. 6 gezeigt ist. Der teleskopartige Verlängerungsabschnitt 18 weist zwei oder mehr koaxial jeweils ineinander liegende zylindrische Teilröhren auf. Die distale, innere Teilröhre (hier der distale Endabschnitt 12 des Arbeitskanals 10) kann aus der äußeren Teilröhre herausfahren. Vorteilhafter Weise ist ein Anschlag vorgesehen, der die Ausfahrbewegung begrenzt (dies kann auch durch eine leichte Konizität erreicht werden). Zudem ist in dem dargestellten Ausführungsbeispiel eine Dichtung zwischen den relativbeweglichen Teilröhren vorgesehen.

Figur 10 zeigt einen alternativen Verlängerungsabschnitt 18 der Wellenschlauch-Bauform. Die Außenhülle des Arbeitskanals 10 ist bei einer solchen Ausführungsform gewellt/gerafft ausgeführt. Bei einer Zugbelastung kann die für die Ausfahrbewegung des distalen Endabschnitts des Arbeitskanals 12 benötigte Schlauch- /Kanallänge aus diesem gewellten / gerafften Bereich geholt werden.

Die vorliegenden Ausführungsformen sind ebenso gut ohne Verlängerungsabschnitt 18 ausführbar, indem die für das Ausfahren erforderliche Arbeitskanallänge bspw. aus dessen Eigenelastizität geholt wird.

Es ist im Rahmen dieser Offenbarung angedacht, Elemente der oben beschriebenen Ausführungsformen zu kombinieren. So könnte bspw. ein Schleppmittel 16 gemäß der ersten Ausführungsform mit einem Formgedächtnismaterialabschnitt 20 gemäß der zweiten Ausführungsform kombiniert werden. Auch ist die Methode der Auflängung (Teleskopabschnitt, Wellenschlauch, gesamtverschieblicher Arbeitskanal etc.) zwischen den Ausführungsformen austauschbar.

### Bezugszeichen

- 1: Arbeitskanaleinrichtung;
- 2: Endoskop;
- 4: Endoskopkopf;
- 5: distaler Endabschnitt / Endkappe
- 6: Optik;
- 7: Reinigungsdüse;
- 8: Leuchtmittel;
- 10: Arbeitskanal;
- 11: Arbeitskanalausgang;
- 12: distaler Endabschnitt des Arbeitskanals;
- 14: Führungseinrichtung / Rampe;
- 16: Schleppmittel;
- 18: Verlängerungsabschnitt / Teleskopabschnitt;
- 20: Führungseinrichtung / Formgedächtnismaterial;
- 22: Führungseinrichtung;
- D: Duodenum;
- P: Papilla Vateri; und
- W: Werkzeug.

## Patentansprüche

1. Endoskopeinrichtung eines Endoskops (2) mit einer Arbeitskanaleinrichtung (1) zur Führung von medizinischen Werkzeugen (W) und/oder zur Durchströmung von Medien bis zu einem distalen Endoskopkopf (4), wobei die Arbeitskanaleinrichtung (1) Folgendes aufweist:
einen zumindest abschnittsweise biegeflexibel und relativ zum Endoskop (2) ausfahrbar ausgebildeten Arbeitskanal (10), der
- von einer ersten Stellung, in der ein distaler Arbeitskanalausgang (11) in Axialrichtung des Endoskopkopfs (4) ausgerichtet ist,
- in eine zweite, gegenüber der ersten Stellung in Richtung distal ausgefahrene Stellung verlagerbar ist, und
eine Führungseinrichtung (14), mittels welcher der Arbeitskanalausgang (11) in der zweiten Stellung in eine vorbestimmte laterale oder rückblickende Richtung umgelenkt oder umlenkbar ist,
**gekennzeichnet durch**
ein von einem Griffteil des Endoskops (2) her betätigbares und im Bereich des Arbeitskanalausgangs (11) verankertes Schleppmittel (16), insbesondere Zugseil, mittels welchem der Arbeitskanal (10) von der ersten in die zweite Stellung schleppbar ist, und
eine einem distalen Arbeitskanalende in der ersten Stellung distal vorgelagerte Umlenkeinrichtung, durch welche das Schleppmittel (16) derart umgelenkt wird, dass der Arbeitskanal (10) beim Aufbringen einer Zugkraft auf das Schleppmittel (16) von einer proximalen Richtung her zunächst gen distal geschleppt wird und anschließend einer Umlenkung des Schleppmittels (16) folgend in eine laterale Ausrichtung gegenüber einer Endoskoplängsachse gezogen wird.

2. Endoskopeinrichtung gemäß Anspruch 1, **dadurch gekennzeichnet, dass** die Führungseinrichtung (14) eine zum distalen Endoskopende hin konvexe Führungsfläche aufweist, insbesondere eine konvex verlaufende Führungsrinne, gegen welche der Arbeitskanal (10) in der zweiten Stellung durch das Schleppmittel (16) gezogen wird, wodurch die Führungseinrichtung (14) den Arbeitskanal (10) von radial innen abstützt und diesem aufgrund seiner Biegeflexibilität eine definierte Krümmung verleiht.

3. Endoskopeinrichtung gemäß einem der vorgenannten Ansprüche 1 und 2, **dadurch gekennzeichnet, dass** die Führungseinrichtung (14) die Umlenkeinrichtung ausbildet.

4. Endoskopeinrichtung mit einer Arbeitskanaleinrichtung (1) zur Führung von medizinischen Werkzeugen (W) und/oder zur Durchströmung von Medien bis zu einem distalen Endoskopkopf (4), wobei die Arbeitskanaleinrichtung Folgendes aufweist:
einem zumindest abschnittsweise biegeflexibel und relativ zum Endoskop (2) ausfahrbar ausgebildeten Arbeitskanal (10), der
- von einer ersten Stellung, in der ein distaler Arbeitskanalausgang (11) in Axialrichtung des Endoskopkopfs (4) ausgerichtet ist,
- in eine zweite, gegenüber der ersten Stellung in Richtung distal ausgefahrene Stellung verlagerbar ist, und
einer Führungseinrichtung, mittels welcher der Arbeitskanalausgang (11) in der zweiten Stellung in eine vorbestimmte laterale oder rückblickende Richtung umgelenkt oder umlenkbar ist,
**gekennzeichnet durch**
einen distalen Endoskopkopfabschnitt (5), welcher in eine laterale Stellung schwenkbar ausgebildet ist, wobei der ausfahrbare Arbeitskanal (10) an besagtem distalen Endoskopkopfabschnitt (5) als einem Schleppmittel fixiert ist, sodass bei einer Schwenkbewegung des distalen Endoskopkopfabschnitts (5) dieser den Arbeitskanal (10) ausfährt und umlenkt.

5. Endoskopeinrichtung gemäß Anspruch 4, **dadurch gekennzeichnet, dass** bei einer Schwenkbewegung des distalen Endoskopkopfabschnitts (5) eine weitere Führungseinrichtung (22) aufklappt, welche den ausgefahrenen Arbeitskanal (10) von radial innen her mit einer definiert gekrümmten Kontur abstützt.

6. Endoskopeinrichtung nach einem der vorstehenden Ansprüche 1 bis 5, **dadurch gekennzeichnet, dass** das Endoskop eine in Axialrichtung blickende Optik (6) und eine zusätzliche lateral blickende Optik (6) aufweist, oder schräg ausgerichtete Optik (6) aufweist oder eine Optik (6) aufweist, die von einer in Axialrichtung blickenden Ausrichtung in eine lateral blickende Ausrichtung überführbar ausgebildet ist.

7. Endoskopeinrichtung gemäß einem der vorgenannten Ansprüche 1 bis 6, **dadurch gekennzeichnet, dass** der Arbeitskanal (10) einen Verlängerungsabschnitt (18), insbesondere einen teleskopierbaren und / oder dehnbaren Abschnitt aufweist, der sich bei einer Zugbelastung des Arbeitskanals (10) in dessen Axialrichtung verlängert und dadurch die Ausfahrbewegung eines distalen Arbeitskanalabschnitts (12) unterstützt.

## Claims

1. An endoscope device of an endoscope (2) having a work channel arrangement (1) for guiding medical tools (W) and/or for the flow of media up to a distal endoscope head (4), wherein the work channel arrangement (1) comprises the following:
a work channel (10) which is designed to be flexurally elastic at least in sections and extendable relative to the endoscope (2) and
- which can be displaced from a first position, in which a distal work channel exit (11) is oriented in the axial direction of the endoscope head (4),
- into a second position extended distally relative to the first position, and
a guiding device (14) via which the work channel exit (11) is deflected or deflectable in a predetermined lateral or retrospective direction in the second position,
**characterized by**
an entrainer (16), in particular a pull cable, which can be operated from a handle part of the endoscope (2) and which is anchored in the area of the work channel exit (11) and via which the work channel (10) can be pulled from the first into the second position, and
a deflection device which is located distally in front of a distal working channel end in the first position and via which the entrainer (16) is deflected in such a way that, when a tensile force is applied to the entrainer (16), the working channel (10) is initially entrained from a proximal direction towards the distal direction and then, following a deflection of the entrainer (16), is drawn into a lateral orientation with respect to an longitudinal endoscope axis.

2. The endoscope device according to claim 1, **characterized in that** the guiding device (14) has a guide surface which is convex towards the distal end of the endoscope, in particular a convexly extending guide groove, wherein the work channel (10) is being pulled against the guide surface (14) in the second position by the entrainer (16), whereby the guiding device (14) supports the work channel (10) from radially inside and gives it a defined curvature due to its flexural elasticity.

3. The endoscope device (2) according to one of the aforementioned claims 1 and 2, **characterized in that** the guiding device (14) forms the deflection device.

4. The endoscope device having a work channel arrangement (1) for guiding medical tools (W) and/or for the flow of media up to a distal endoscope head (4), wherein the work channel arrangement (1) comprises the following:
a work channel (10) which is designed to be flexurally elastic at least in sections and extendable relative to the endoscope (2) and
- which can be displaced from a first position, in which a distal work channel exit (11) is oriented in the axial direction of the endoscope head (4),
- into a second position extended distally relative to the first position, and
a guiding device via which the work channel exit (11) is deflected or deflectable in a predetermined lateral or retrospective direction in the second position,
**characterized by**
a distal endoscope head portion (5) which is designed to be pivotable into a lateral position and the extendable work channel (10) is fixed to said distal endoscope head portion (5) as an entrainer, so that when the distal endoscope head portion (5) performs a pivoting movement, it extends and deflects the work channel (10).

5. The endoscope device according to claim 4, **characterized in that** during a pivoting movement of the distal endoscope head portion (5), a further guiding device (22) folds open, which supports the extended work channel (10) from radially inside with a defined curved contour.

6. The endoscope device according to one of the preceding claims 1 to 5, **characterized in that** the endoscope has an optical unit (6) facing in the axial direction and an additional optical unit (6) facing in the lateral direction, or has an obliquely oriented optical unit (6), or comprises an optical unit (6) that is designed so that it can be transferred from an orientation facing in the axial direction to an orientation facing in the lateral direction.

7. The endoscope device according to one of the aforementioned claims 1 to 6, **characterized in that** the working channel (10) has an extension section (18), in particular a telescopic and/or extensible section, which extends in the axial direction of the working channel (10) when the latter is subjected to a tensile load and thereby supports the extension movement of a distal working channel section (12).

## Revendications

1. Dispositif endoscopique d'un endoscope (2) avec un dispositif de canal de travail (1) pour le guidage d'outils médicaux (W) et/ou pour le passage de fluides jusqu'à une tête d'endoscope distale (4), dans lequel le dispositif de canal de travail (1) présente les éléments suivants :
un canal de travail (10) flexible en flexion au moins par sections et réalisé de façon à pouvoir être déployé par rapport à l'endoscope (2), lequel
- est réalisé à partir d'une première position dans laquelle une sortie distale du canal de travail (11) est orientée dans la direction axiale de la tête d'endoscope (4),
- peut être déplacé dans une seconde position, étendue en direction distale par rapport à la première position, et
un dispositif de guidage (14), au moyen duquel la sortie de canal de travail (11) est déviée ou peut être déviée dans la seconde position dans une direction latérale ou rétrograde prédéterminée,
**caractérisé par**
un moyen de remorquage (16), en particulier un câble de traction, pouvant être actionné à partir d'une partie de préhension de l'endoscope (2) et ancré dans la zone de la sortie de canal de travail (11), au moyen duquel le canal de travail (10) peut être remorqué de la première à la seconde position, et
un dispositif de déviation placé de manière distale devant une extrémité distale du canal de travail dans la première position, par lequel le moyen de remorquage (16) est dévié de sorte que le canal de travail (10), lors de l'application d'une force de traction sur le moyen de remorquage (16), est d'abord remorqué à partir d'une direction proximale vers la direction distale et est ensuite tiré dans une orientation latérale par rapport à un axe longitudinal d'endoscope, en suivant une déviation du moyen de remorquage (16).

2. Dispositif endoscopique selon la revendication 1, **caractérisé en ce que** le dispositif de guidage (14) présente une surface de guidage convexe vers l'extrémité distale de l'endoscope, en particulier une goulotte de guidage s'étendant de manière convexe, contre laquelle le canal de travail (10) est tiré dans la seconde position par le moyen de remorquage (16), moyennant quoi le dispositif de guidage (14) soutient le canal de travail (10) radialement à partir de l'intérieur et lui confère une courbure définie en raison de sa flexibilité à la flexion.

3. Dispositif endoscopique selon l'une quelconque des revendications 1 et 2 précitées, **caractérisé en ce que** le dispositif de guidage (14) forme le dispositif de déviation.

4. Dispositif endoscopique avec un dispositif de canal de travail (1) pour le guidage d'outils médicaux (W) et/ou pour le passage de fluides jusqu'à une tête d'endoscope distale (4), dans lequel le dispositif de canal de travail présente les éléments suivants :
un canal de travail (10) flexible en flexion au moins par sections et réalisé de façon à pouvoir être déployé par rapport à l'endoscope (2), lequel
- est réalisé à partir d'une première position dans laquelle une sortie distale du canal de travail (11) est orientée dans la direction axiale de la tête d'endoscope (4),
- peut être déplacé dans une seconde position, étendue en direction distale par rapport à la première position, et
un dispositif de guidage, au moyen duquel la sortie de canal de travail (11) est déviée ou peut être déviée dans la seconde position dans une direction latérale ou rétrograde prédéterminée,
**caractérisé par**
une section de tête d'endoscope distale (5) qui est réalisée de manière à pouvoir pivoter dans une position latérale, dans lequel le canal de travail (10) pouvant être déployé est fixé à ladite section de tête d'endoscope distale (5) en tant que moyen de remorquage, de sorte que lors d'un mouvement de pivotement de la section de tête d'endoscope distale (5), celle-ci déploie et dévie le canal de travail (10).

5. Dispositif endoscopique selon la revendication 4, **caractérisé en ce que**, lors d'un mouvement de pivotement de la partie distale (5) de la tête d'endoscope, un autre dispositif de guidage (22) s'ouvre, lequel soutient le canal de travail (10) déployé radialement à partir de l'intérieur avec un contour à courbure définie.

6. Dispositif endoscopique selon l'une quelconque des revendications 1 à 5 ci-dessus, **caractérisé en ce que** l'endoscope présente une optique (6) orientée dans une direction axiale et une optique (6) supplémentaire orientée latéralement, ou présente une optique (6) orientée obliquement ou présente une optique (6) qui est réalisée de manière à pouvoir passer d'une orientation orientée dans une direction axiale à une orientation orientée latéralement.

7. Dispositif endoscopique selon l'une quelconque des revendications 1 à 6 précitées, **caractérisé en ce que** le canal de travail (10) présente une section de prolongement (18), en particulier une section télescopique et/ou extensible, qui s'allonge dans la direction axiale du canal de travail (10) lors d'une sollicitation en traction de ce dernier et assiste ainsi le mouvement de sortie d'une section distale (12) de canal de travail.
